(19) Europäisches Patentamt
European Patent Office
Office européen des brevets

(11) EP 0 321 054 B1

(12) EUROPEAN PATENT SPECIFICATION

(45) Date of publication and mention of the grant of the patent:
**06.03.1996 Bulletin 1996/10**

(51) Int Cl.6: **C07C 69/24**, C07C 67/38, C07C 67/03

(21) Application number: **88202870.7**

(22) Date of filing: **13.12.1988**

# (54) Process for the production of propionate esters

Herstellungsverfahren von Propionsäureestern

Procédé de production d'esters de l'acide propionique

(84) Designated Contracting States:
**BE DE FR GB IT NL**

(30) Priority: **15.12.1987 GB 8729267**

(43) Date of publication of application:
**21.06.1989 Bulletin 1989/25**

(73) Proprietor: **SHELL INTERNATIONALE RESEARCH MAATSCHAPPIJ B.V.**
**NL-2596 HR Den Haag (NL)**

(72) Inventors:
- **Petrus, Leonardus**
  **NL-1031 CM Amsterdam (NL)**
- **De Bruijn, Wouter**
  **NL-2596 HR The Hague (NL)**

(56) References cited:
**EP-A- 0 017 441** **EP-A- 0 235 864**
**US-A- 3 098 093**

Remarks:
The file contains technical information submitted after the application was filed and not included in this specification

EP 0 321 054 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The invention relates to a process for the production of propionate esters.

Propionate esters can be produced by carbonylation of ethylene with carbon monoxide in the presence of an alcohol and a carbonylation catalyst according to reaction equation A

$$CH_2 = CH_2 + CO + ROH \rightarrow CH_3CH_2C(O)OR \qquad (A)$$

in which R represents an organic group (see for example EP-A-235 864). In this manner, methyl propionate can be produced starting from methanol as the alcohol ROH. Simultaneous production of methyl propionate and the propionate ester of an alcohol, other than methanol, may take place by carrying out two such carbonylation processes, one starting from methanol and the other starting from the alcohol other than methanol.

An integrated process for the simultaneous production of methyl propionate and the second propionate ester of an alcohol other than methanol has now been found which is considerably simpler than the total of two such carbonylation processes.

Accordingly, the invention provides a process for the production of propionate esters which process comprises the simultaneous production of methyl propionate and the propionate ester of an alcohol other than methanol by means of the following steps:-

step 1: carbonylating ethylene with carbon monoxide in the presence of methanol and a carbonylation catalyst with formation of methyl propionate;

step 2: isolating methyl propionate from the reaction mixture obtained in step 1;

step 3: transesterifying methyl propionate by reaction with the alcohol other than methanol in the presence of a transesterification catalyst;

step 4: isolating the propionate ester of said alcohol other than methanol and an azeotrope comprising methanol and methyl propionate by means of distillation from the reaction mixture obtained in step 3; and

step 5: recirculating the azeotrope isolated in step 4 to the carbonylation in step 1.

As will be more fully described hereinafter the simplicity of the process according to the present invention resides in the fact that the methanol-methyl propionate azeotrope can very easily be isolated in step 4 and re-used for reaction in the carbonylation of step 1, irrespective of which alcohol other than methanol is used in step 3. The "alcohol other than methanol" is also referred to hereinafter as "second alcohol". The "propionate ester of the alcohol other than methanol" is also referred to hereinafter as "second ester".

The methyl propionate for step 3 may originate from any source, but it is a preferred feature of the present invention that this methyl propionate has been isolated in step 1. The carbonylation in step 1 involves the reaction of ethylene with carbon monoxide and methanol with formation of methyl propionate. The reaction mixture obtained in step 1 therefore contains methyl propionate, methanol, carbon monoxide, ethylene and carbonylation catalyst. According to a preferred embodiment of the present invention step 2 is carried out by separating the liquid phase obtained after gas-liquid separation of the reaction mixture obtained in step 1 by means of distillation into a lighter fraction comprising an azeotrope of methanol and methyl propionate, a heavier fraction comprising methyl propionate, and a residual fraction containing the carbonylation catalyst, the term "heavier" meaning that the boiling point of this fraction is higher than that of said lighter fraction. The gas-liquid separation is what is usually referred to as a "flash" and is therefore carried out at such a temperature and pressure that the gaseous phase which is flashed off substantially consists of carbon monoxide and ethylene. Said azeotrope contains about 50% by weight of methanol and about 50% by weight of methyl propionate and has a boiling point at atmospheric pressure of 62°C. In this manner, a heavier fraction having a methyl propionate content of more than 99.9% by weight can be obtained. The lighter fraction can be given any desired destination but is preferably recirculated to the carbonylation in step 1 so as to re-use the methanol present in it for the production of further quantities of methyl propionate, thereby simultaneously separating the azeotrope into its two components. Suitably, the distillation of step 2 may take place in a first column in which a non-azeotropic top fraction comprising methanol and methyl propionate, and said residual fraction containing the carbonylation catalyst are obtained, and a second column in which the top fraction from the first column is separated into a top fraction comprising the azeotrope (which is the lighter fraction mentioned hereinbefore) and said heavier fraction comprising methyl propionate. Said residual fraction containing the carbonylation catalyst and also some methyl propionate can be given any desired destination but is preferably recirculated to the carbonylation in step 1 so as to carbonylate further quantities of ethylene.

According to another embodiment of the present invention step 2 is carried out by separating the reaction mixture obtained in step 1 after flashing into a gaseous fraction comprising methanol and methyl propionate, and a liquid fraction containing the carbonylation catalyst. The gaseous fraction is condensed and the condensate thus obtained separated by means of distillation into (a) a top fraction comprising an azeotrope of methanol and methyl propionate which azeotrope may be recirculated to the carbonylation in step 1, (b) an intermediate fraction comprising methyl propionate which may have a purity of more than 99.9 % by weight, and (c) a relatively small residual fraction. The liquid fraction containing the carbonylation catalyst may be recirculated to the carbonylation in step 1.

The reaction mixture obtained in step 3 contains the second ester, methanol, methyl propionate, the second alcohol and the transesterification catalyst, transesterification being an equilibrium reaction, and is distilled in step 4 so as to isolate an azeotrope of methanol and methyl propionate. The process according to the present invention excels in simplicity because the recirculation of this azeotrope to the carbonylation of step 1 involves simultaneous separation thereof into its components, the separated methanol being used for the production of further quantities of methyl propionate in step 1 and the separated methyl propionate finally becoming available in step 2.

Amending the process according to the present invention by replacing step 5 with recirculation of the azeotrope to the transesterification of step 3 would result in shifting the equilibrium reaction of the transesterification

$$\text{methyl propionate} + \text{second alcohol} \rightleftarrows \text{second ester} + \text{methanol}$$

to the left, this azeotrope containing methanol and methyl propionate in a molar ratio of about 3:1.

The distillation in step 4 is preferably carried out so as to yield an intermediate fraction comprising alcohol other than methanol (the second alcohol), an intermediate fraction comprising the propionate ester of said alcohol other than methanol (the second ester) and a residual fraction comprising the transesterification catalyst. The intermediate fraction comprising the second ester, may have a purity of more than 99.9% by weight. The intermediate fraction, comprising the second alcohol, and the residual fraction comprising the transesterification catalyst may be given any desired destination, but are preferably recirculated to the transesterification of step 3.

A wide variety of alcohols other than methanol may be used in the transesterification of step 3. The alcohols may be aliphatic, cycloaliphatic or aromatic and may be substituted with one or more substituents, for example with one or more halogen atoms or cyano, ester, alkoxy, carboxy or aryl groups. The alcohol may therefore also be a phenol. The alcohols preferably do not contain more than 20 carbon atoms per molecule and, in particular, have in the range of from 2 to 10 carbon atoms per molecule. Alkanols are particularly preferred; among these, very good results have been obtained with 2-butanol. Other examples of suitable alcohols are ethanol, propanol, 2-propanol, butanol, tert.-butyl alcohol, pentanol, 2-pentanol, 2-methylbutanol, 2-methylpentanol, hexanol, heptanol, octanol, nonanol, decanol, stearyl alcohol, benzyl alcohol, cyclohexanol, chlorocapryl alcohol and phenethyl alcohol. The alcohol may be polyhydric, examples of which are ethylene glycol, 1,2-propanediol, 1,4-butanediol, 1,3-butanediol, glycerol, pentaerythritol, pinacol, 1,6-hexanediol and polyethylene glycol. Other examples are phenol, o-, m-and p-cresol, 2-naphthol and 9-anthrol.

Step 1 of the process according to the present invention can be carried out in the presence of any carbonylation catalyst. Preference is given to carbonylation catalysts which have been prepared by combining:-

(a) a palladium compound,
(b) at least 5 mol of a phosphine having the general formula I

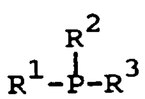

(I)

in which $R^1$, $R^2$ and $R^3$ each represent an optionally substituted aryl group, per gram-atom of palladium, and
(c) a protonic acid having a $pK_a$ below 2, measured at 18°C in aqueous solution, except hydrohalogenic acids and carboxylic acids.

Such carbonylation catalysts have been described in EP-A-106,379.

Application of these carbonylation catalysts results in a very high reaction rate. Both homogeneous and heterogeneous carbonylation catalysts may be used, but homogeneous carbonylation catalysts are preferred.

Step 1 may be carried out using a molar ratio ethylene to methanol which is not critical and may vary within wide ranges, preferably between 0.1:1 to 10:1, in particular 0.2:1 to 1:1.

The carbon monoxide to be used in step 1 may be used pure or diluted with an inert gas, such as nitrogen, noble gases or carbon dioxide. Generally, the presence of more than 10% by volume of hydrogen is undesirable, since under the carbonylation conditions it may cause hydrogenation of the ethylene. Generally, preference is given to the use of carbon monoxide or a gas containing carbon monoxide which contains less than 5% by volume of hydrogen.

A great variety of transesterification catalysts may be used in step 3 of the process according to the present invention. Such catalysts may also be referred to as "alcoholysis catalysts", and may be acid or basic. Suitable catalysts include sulphuric acid, orthophosphoric acid, p-toluenesulphonic acid, ethyl hydrogen sulphate, ethanesulphuric acid, aluminium chloride, zinc chloride, sodium isobutoxide, aluminium isobutoxide and methanesulphonic acid.

The transesterification in step 3 may be carried out using a molar ratio transesterification catalyst to methyl propionate, a molar ratio second alcohol to methyl propionate and at a temperature and pressure which are not critical and may vary within wide ranges. Preferably, a molar ratio transesterification catalyst to methyl propionate in the range of

from 1:20 to 1:1000, a molar ratio second alcohol to methyl propionate in the range of from 1:0.2 to 1:5, a temperature in the range of from 50°C to 150°C and a pressure in the range of from 1 to 10 bar are applied.

Example

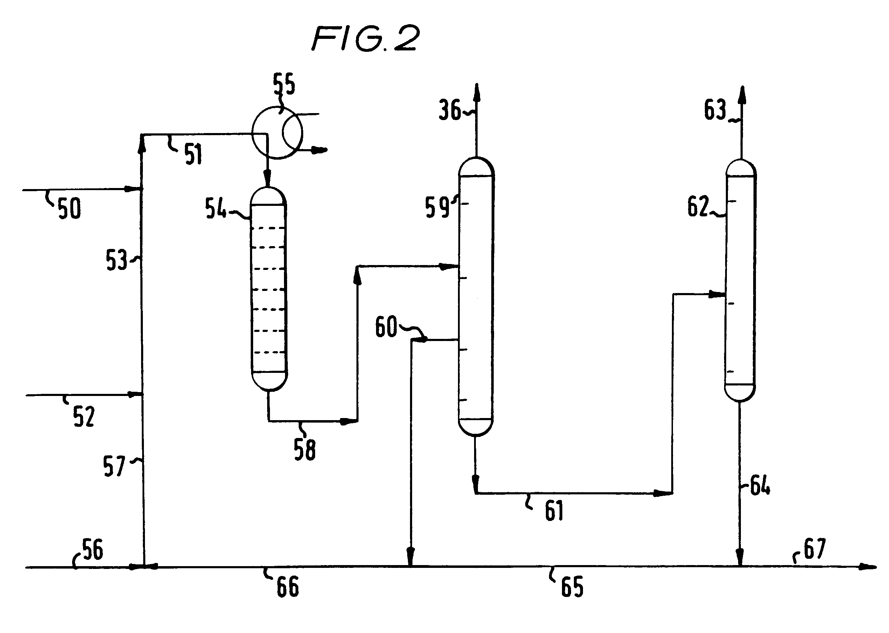

The invention will now be described in more detail by way of example with reference to the accompanying drawings, wherein Figure 1 shows a simplified process flow scheme of steps 1, 2 and 5 and Figure 2 of steps 3 and 4 of the process according to the present invention.

Referring to Figure 1, carbon monoxide is supplied via a line 1, a line 2 and a line 3, ethylene via a line 4 and the line 3, methanol via a line 5 and a line 6 and a catalytic system via a line 7, a line 8 and the line 6 to a reactor 9. Compressed recycle gas is conducted via a line 10 and the lines 2 and 3 to the reactor 9. A recycle of azeotrope is conducted via a line 11 to the line 8. The reactor 9 is provided with a stirrer 12 and cooling means 13. The temperature in the reactor is 110°C, the pressure is 40 bar and the residence time of the reaction mixture 2 hours.

The reaction mixture formed in the reactor 9 (step 1) is withdrawn therefrom via a line 14 and introduced into a flash vessel 15 in which it is separated into a liquid phase and a gaseous phase, withdrawn from the flash vessel 15 via a line 16 and a line 17, respectively. The liquid phase conducted through the line 16 is further conducted through a line 18 and then introduced into a distillation column 19 in which it is separated into a non-azeotropic top fraction comprising methanol and methyl propionate and a residual fraction containing the carbonylation catalyst, which are withdrawn from the column 19 via a line 20 and a line 21, respectively. The non-azeotropic top fraction conducted through the line 20 is separated by condensation in a condenser 22 into a condensate and a gas fraction which are withdrawn from the condenser 22 via a line 23 and a line 24, respectively. A portion of the condensate conducted through the line 23 is returned as reflux to the distillation column 19 via a line 25; the balance is conducted via a line 26 to a distillation column 27 in which it is separated into a top fraction comprising an azeotrope of methanol and methyl propionate and a heavier fraction comprising methyl propionate, withdrawn from the column 27 via a line 28 and a line 29, respectively. The heavier fraction in line 29 has a methyl propionate content of more than 99.9% by weight. A portion of this methyl propionate is fed to steps 3 and 4 which are further discussed hereinafter with respect to Figure 2.

The top fraction conducted through the line 28 is separated by condensation in a condenser 30 in a condensate comprising an azeotrope of methanol and methyl propionate and a gas fraction, which are withdrawn from the condenser 30 via a line 31 and a line 32, respectively. A portion of the condensate conducted through the line 31 is returned as reflux to the distillation column 27 via a line 33 and the balance thereof is returned via a line 34, a line 35 and the line 11 to the line 8.

An azeotropic mixture of methanol and methyl propionate originating from steps 3 and 4 described hereinafter in connection with Figure 2 is introduced into the line 11 via a line 36.

The gases conducted via the line 32 are conducted via a line 38, a line 39 and a line 40, those from the line 24 via the lines 39 and 40 and those from the line 17 via the line 40 to a compressor 41. The gases compressed in the compressor 41 become available as the compressed recycle gas mentioned hereinbefore and conducted via the line 10.

The residual fraction containing the carbonylation catalyst and conducted via the line 21 is partly recycled via a line 42 to the reactor 9 and partly conducted via a line 43 to a stripper 44 in which methyl propionate is stripped off and conducted via a line 45 to the line 18, leaving a small residue of heavy ends which is withdrawn from the stripper 44 via a line 46.

Bleed streams are withdrawn from the line 38 via a line 47 and from the line 34 via a line 48.

The following material balance applies to Figure 1:

| In | Line | Amount kg/h | Out | Line | Amount kg/h |
|---|---|---|---|---|---|
| | 1 | 2.000 | | 29 | 6.903 |
| | 4 | 2.003 | | 46 | 0.094 |
| | 5 | 1.662 | | 47 | 0.090 |
| | 36 | 1.382 | | 48 | 0.003 |
| | 7 | 0.043 | | | |
| | | 7.090 | | | 7.090 |

Referring to Figure 2, methyl propionate is supplied via a line 50 and a line 51 and 2-butanol via a line 52, a line 53 and the line 51 to a reactor 54. The methyl propionate in line 50 originates from the line 29 depicted in Figure 1 and, therefore, has been produced according to steps 1 and 2. The balance of the methyl propionate from line 29 has been given another destination. The mixture of methyl propionate and 2-butanol is heated in a heater 55 prior to entering the reactor 54. The transesterification catalyst is supplied via a line 56 and a line 57 to the line 53. The reactor 54 is operated

in plug-flow, at a temperature of 90° C and a pressure of 6 bar.

The reaction mixture formed in the reactor 54 (step 3) is withdrawn therefrom via a line 58 and introduced into a distillation column 59 in which it is separated into a top fraction, withdrawn from the column 59 via the line 36, an intermediate fraction withdrawn via a line 60 and a bottom fraction withdrawn via a line 61. The top fraction is the azeotrope comprising methanol and methyl propionate isolated according to step 4, line 36 in Figure 2 being the same line as line 36 in Figure 1.

The bottom fraction withdrawn via the line 61 is introduced into a distillation column 62 in which it is separated into a top fraction having a content of sec-butyl propionate of more than 99.9% by weight and withdrawn via a line 63, and a bottom fraction withdrawn via a line 64. This bottom fraction is further conducted via a line 65 and a line 66 to the line 57 and the intermediate fraction from the line 60 is introduced into the line 66. A bleed stream is withdrawn from the line 64 via a line 67.

The following material balance applies to Figure 2:

| In | Line | kg/h | Out | Line | kg/h |
|---|---|---|---|---|---|
| | 50 | 2.483 | | 63 | 2.540 |
| | 52 | 1.446 | | 36 | 1.382 |
| | 56 | 0.008 | | 67 | 0.015 |
| | | 3.937 | | | 3.937 |

Comparative Experiment

The experiment described in the Example with respect to Figure 1 was repeated with the difference that the supply of the azeotrope via the line 36 was stopped. Then, the following material balance applies to Figure 1:

| In | Line | Amount kg/h | Out | Line | Amount kg/h |
|---|---|---|---|---|---|
| | 1 | 2.000 | | 29 | 6.133 |
| | 4 | 2.003 | | 46 | 0.094 |
| | 5 | 2.284 | | 47 | 0.090 |
| | 36 | 0 | | 48 | 0.003 |
| | 7 | 0.043 | | | |
| | | 6.330 | | | 6.330 |

**Claims**

**1.** A process for the production of propionate esters which process comprises the simultaneous production of methyl propionate and the propionate ester of an alcohol other than methanol by means of the following steps:-

step 1: carbonylating ethylene with carbon monoxide in the presence of methanol and a carbonylation catalyst with formation of methyl propionate;
step 2: isolating methyl propionate from the reaction mixture obtained in step 1;
step 3: transesterifying methyl propionate by reaction with the alcohol other than methanol in the presence of a transesterification catalyst;
step 4: isolating the propionate ester of said alcohol other than methanol and an azeotrope comprising methanol and methyl propionate by means of distillation from the reaction mixture obtained in step 3; and
step 5: recirculating the azeotrope isolated in step 4 to the carbonylation in step 1.

**2.** A process as claimed in claim 1 in which the methyl propionate to be transesterified in step 3 has been isolated in step 2.

**3.** A process as claimed in claim 1 or 2 in which step 2 is carried out by separating the liquid phase obtained after gas-liquid separation of the reaction mixture obtained in step 1 by means of distillation into a lighter fraction comprising an azeotrope of methanol and methyl propionate, a heavier fraction comprising methyl propionate, and a residual fraction containing the carbonylation catalyst.

**4.** A process as claimed in claim 3 in which the lighter fraction comprising the azeotrope is recirculated to the carbonylation in step 1.

**5.** A process as claimed in any one of the preceding claims in which the distillation in step 4 also yields an intermediate fraction comprising alcohol other than methanol, an intermediate fraction comprising the propionate ester of said alcohol other than methanol and a residual fraction comprising the transesterification catalyst.

**6.** A process as claimed in claim 5 in which the intermediate fraction comprising alcohol other than methanol and the bottom fraction comprising the transesterification catalyst are recirculated to the transesterification of step 3.

**7.** A process as claimed in any one of the preceding claims in which the carbonylation catalyst being used in step 1 has been prepared by combining

(a) a palladium compound,
(b) at least 5 mol of a phosphine having the general formula I

$$\begin{array}{c} R^2 \\ | \\ R^1-P-R^3 \end{array} \qquad (I)$$

in which $R^1$ , $R^2$ and $R^3$ each represent an optionally substituted aryl group, per gram-atom of palladium, and
(c) a protonic acid having a $pK_a$ below 2, measured at 18 °C in aqueous solution, except hydrohalogenic acids and carboxylic acid.

**8.** A process as claimed in any one of the preceding claims in which the alcohol other than methanol being used in step 3 has in the range of from 2 to 10 carbon atoms per molecule.

**9.** A process as claimed in claim 8 in which the alcohol is an alkanol.

**10.** A process as claimed in claim 9 in which the alcohol is 2-butanol.

**Patentansprüche**

**1.** Verfahren zur Herstellung von Propionsäureestern, umfassend die gleichzeitige Herstellung von Propionsäuremethylester und einem Propionsäureester eines von Methanol verschiedenen Alkohols mittels folgender Schritte:-

Schritt 1: Carbonylierung von Ethylen mit Kohlenmonoxid in Gegenwart von Methanol und einem Carbonylierungskatalysator unter Bildung von Propionsäuremethylester,
Schritt 2: Abtrennung von Propionsäuremethylester aus der in Schritt 1 erhaltenen Reaktionsmischung,
Schritt 3: Umesterung von Propionsäuremethylester durch Umsetzung mit einem von Methanol verschiedenen Alkohol in Gegenwart eines Umesterungskatalysators,
Schritt 4: Abtrennung des Propionsäureesters des von Methanol verschiedenen Alkohols und eines Azeotrops aus Methanol und Propionsäuremethylester durch Destillation aus der in Schritt 3 erhaltenen Reaktionsmischung und
Schritt 5: Rückführung des in Schritt 4 abgetrennten Azeotrops zur Carbonylierung in Schritt 1.

**2.** Verfahren nach Anspruch 1, bei dem man den in Schritt 3 umzuesternden Propionsäuremethylester in Schritt 2 abgetrennt hat.

**3.** Verfahren nach Anspruch 1 oder 2, bei dem man Schritt 2 so durchführt, daß man die nach der Gas-Flüssig-Trennung der in Schritt 1 erhaltenen Reaktionsmischung erhaltene Flüssigphase durch Destillation in eine leichtere, ein Azeotrop aus Methanol und Propionsäuremethylester enthaltende Fraktion, eine schwerere, Propionsäuremethylester enthaltende Fraktion und eine den Carbonylierungskatalysator enthaltende Sumpffraktion auftrennt.

**4.** Verfahren nach Anspruch 3, bei dem man die das Azeotrop enthaltende, leichtere Fraktion zur Carbonylierung im Schritt 1 zurückführt.

**5.** Verfahren nach einem der vorhergehenden Ansprüche, bei dem man durch die Destillation im Schritt 4 zusätzlich noch eine den von Methanol verschiedenen Alkohol enthaltende Zwischenfraktion, eine den Propionsäureester des

von Methanol verschiedenen Alkohols enthaltende Zwischenfraktion und eine den Umesterungskatalysator enthaltende Sumpf fraktion erhält.

6. Verfahren nach Anspruch 5, bei dem man die den von Methanol verschiedenen Alkohol enthaltende Zwischenfraktion und die den Umesterungskatalysator enthaltende Sumpf fraktion zur Umesterung des Schritts 3 zurückführt.

7. Verfahren nach einem der vorhergehenden Ansprüche, bei dem man den in Schritt 1 verwendeten Carbonylierungskatalysator durch Kombination

   (a) einer Palladiumverbindung,
   (b) mindestens 5 mol pro Grammatom Palladium eines Phosphins der allgemeinen Formel I

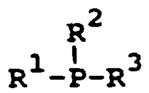

(I) ,

   worin $R^1$, $R^2$ und $R^3$ jeweils eine gegebenenfalls substituierte Arylgruppe darstellen, und
   (c) einer protonischen Säure mit einem $pK_a$.-Wert kleiner 2, gemessen bei 18°C in wäßriger Lösung, mit Ausnahme von Halogenwasserstoffsäuren und Carbonsäure

   herstellt.

8. Verfahren nach einem der vorhergehenden Ansprüche, bei dem man in Schritt 3 als von Methanol verschiedenen Alkohol einen Alkohol mit 2 bis 10 Kohlenstoffatomen pro Molekül einsetzt.

9. Verfahren nach Anspruch 8, bei dem man als Alkohol ein Alkanol einsetzt.

10. Verfahren nach Anspruch 9, bei dem man als Alkohol 2-Butanol einsetzt.

## Revendications

1. Procédé de production d'esters du type propionate, caractérisé en ce qu'il comprend la production simultanée de propionate de méthyle et de l'ester du type propionate d'un alcool autre que le méthanol, par l'intermédiaire des étapes suivantes :

   étape 1: carbonylation de l'éthylène avec le monoxyde de carbone en présence de méthanol et d'un catalyseur de carbonylation avec formation de propionate de méthyle,
   étape 2: isolement du propionate de méthyle du mélange réactionnel obtenu lors de la mise en oeuvre de l'étape 1,
   étape 3: transestérification du propionate de méthyle par réaction avec l'alcool autre que le méthanol en présence d'un catalyseur de transestérification,
   étape 4: isolement du propionate dudit alcool autre que le méthanol et d'un azéotrope comprenant du méthanol et du propionate de méthyle par distillation du mélange réactionnel obtenu au cours de la mise en oeuvre de l'étape 3 et
   étape 5: recyclage de l'azéotrope isolé au cours de la mise en oeuvre de l'étape 4 à la carbonylation réalisée dans l'étape 1.

2. Procédé suivant la revendication 1, caractérisé en ce que le propionate de méthyle à transestérifier dans l'étape 3 a été isolé dans l'étape 2.

3. Procédé suivant la revendication 1 ou 2, caractérisé en ce que l'on entreprend l'étape 2 en séparant la phase liquide obtenue après la séparation gaz-liquide du mélange réactionnel obtenu dans l'étape 1 par l'intermédiaire d'une distillation en une fraction légère comprenant un azéotrope de méthanol et de propionate de méthyle, une fraction plus lourde comprenant du propionate de méthyle et une fraction résiduelle contenant le catalyseur de carbonylation.

4. Procédé suivant la revendication 3, caractérisé en ce que la fraction plus légère comprenant l'azéotrope est recyclée à la carbonylation de l'étape 1.

**5.** Procédé suivant l'une quelconque des revendications précédentes, caractérisé en ce que la distillation dans l'étape 4 engendre également une fraction intermédiaire comprenant un alcool autre que le méthanol, une fraction intermédiaire comprenant l'ester du type propionate dudit alcool autre que le méthanol et une fraction résiduelle comprenant le catalyseur de transestérification.

**6.** Procédé suivant la revendication 5, caractérisé en ce que l'on recycle la fraction intermédiaire comprenant l'alcool autre que le méthanol et la fraction de fond comprenant le catalyseur de transestérification à la transestérification de l'étape 3.

**7.** Procédé suivant l'une quelconque des revendications précédentes, caractérisé en ce que le catalyseur de carbonylation utilisé dans l'étape 1 a été préparé en combinant :

(a) un composé du palladium,
(b) au moins 5 moles d'une phosphine répondant à la formule générale I

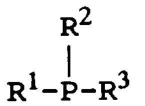 (I)

dans laquelle $R^1$, $R^2$ et $R^3$ représentent chacun un radical aryle éventuellement substitué, par atome-gramme de palladium et
(c) un acide protonique possédant un $pK_a$ inférieur à 2, mesuré à 18°C et en solution aqueuse, à l'exception des acides halogénhydriques et des acides carboxyliques.

**8.** Procédé suivant l'une quelconque des revendications précédentes, caractérisé en ce que l'alcool autre que le méthanol utilisé dans l'étape 3 comporte de 2 à 10 atomes de carbone par molécule.

**9.** Procédé suivant la revendication 8, caractérisé en ce que l'alcool est un alcanol.

**10.** Procédé suivant la revendication 9, caractérisé en ce que l'alcool est le 2-butanol.

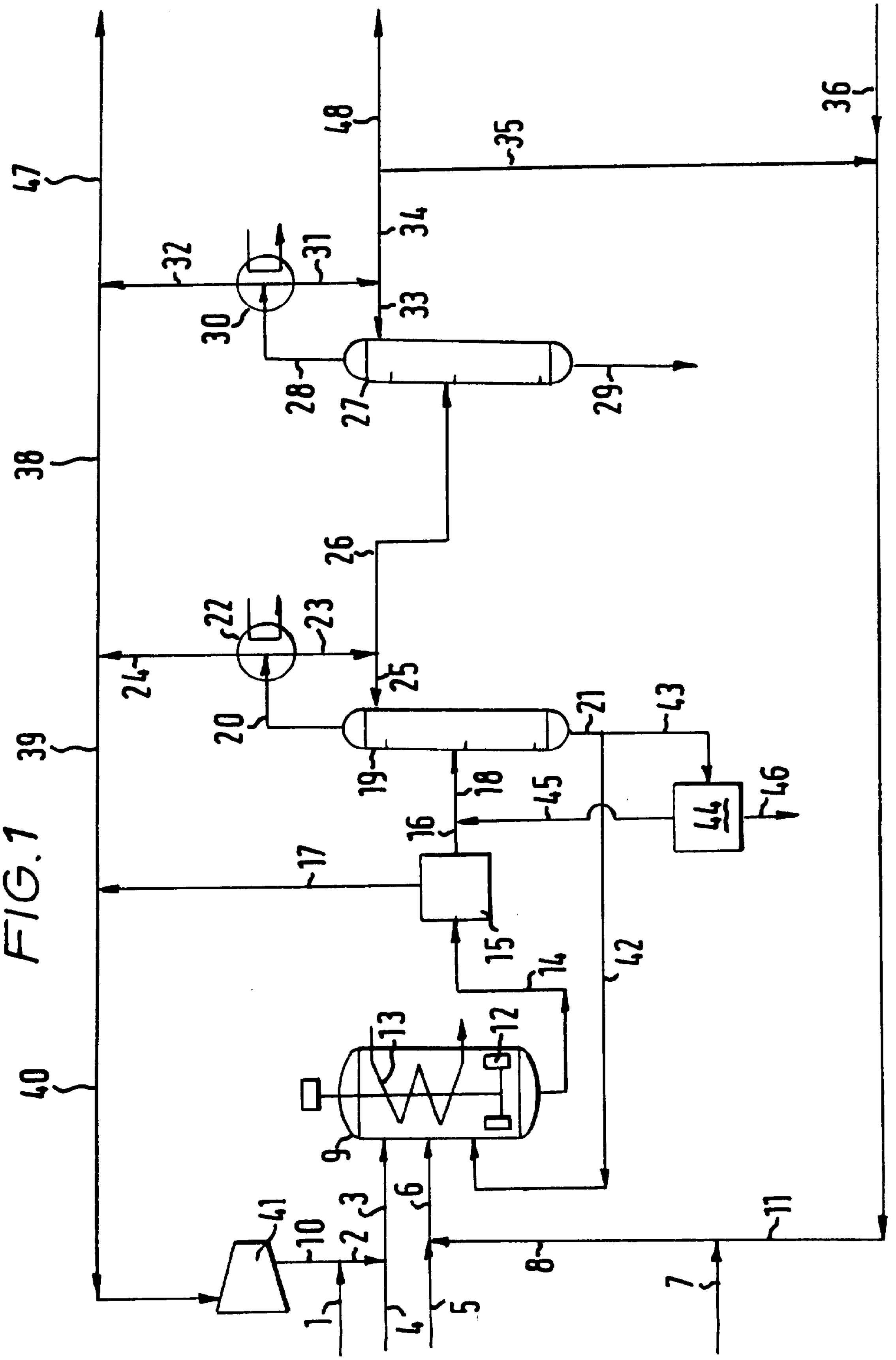
1
2
3
4
5
6
7
8
9
10
11
12
13
14
15
16
17
18
19
20
21
22
23
24
25
26
27
28
29
30
31
32
33
34
35
36
38
39
40
41
42
43
44
45
46
47
48

FIG.1

FIG.2